(19) Europäisches Patentamt
European Patent Office
Office européen des brevets

(11) Veröffentlichungsnummer: **0 017 866**
**B1**

# (12) EUROPÄISCHE PATENTSCHRIFT

(45) Veröffentlichungstag der Patentschrift: **20.10.82**

(51) Int. Cl.³: **C 07 C 53/02**, C 07 C 51/42 // C07C27/02

(21) Anmeldenummer: **80101788.0**

(22) Anmeldetag: **03.04.80**

## (54) Verfahren zur Gewinnung von wasserfreier oder weitgehend wasserfreier Ameisensäure.

(30) Priorität: **11.04.79 DE 2914671**

(43) Veröffentlichungstag der Anmeldung: **29.10.80 Patentblatt 80/22**

(45) Bekanntmachung des Hinweises auf die Patenterteilung: **20.10.82 Patentblatt 82/42**

(84) Benannte Vertragsstaaten: **AT BE CH DE FR GB IT LI NL SE**

(56) Entgegenhaltungen:

**DE-A-2 545 658**
**US-A-2 160 064**

(73) Patentinhaber: **BASF Aktiengesellschaft, Carl-Bosch-Strasse 38, D-6700 Ludwigshafen (DE)**

(72) Erfinder: **Wolf, Dieter, Dr., Auf der Wart 11, D-6718 Gruenstadt 1 (DE)**
Erfinder: **Schmidt, Rudolf, Dr., Paul-Klee-Strasse 1, D-6710 Frankenthal (DE)**
Erfinder: **Block, Ulrich, Dr., Ungsteiner Strasse 14, D-6700 Ludwigshafen (DE)**
Erfinder: **Schoenmakers, Hartmut, Dr., Breslauer Strasse 8, D-6900 Heidelberg (DE)**
Erfinder: **Bott, Kaspar, Dr., Rieslingweg 4, D-6706 Wachenheim (DE)**
Erfinder: **Kaibel, Gerd, Robert-Bosch-Strasse 4, D-6840 Lampertheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 017 866 B1

ACTORUM AG

Verfahren zur Gewinnung von wasserfreier oder weitgehend wasserfreier Ameisensäure

Die vorliegende Erfindung betrifft ein neues Verfahren zur Gewinnung von wasserfreier oder weitgehend wasserfreier Ameisensäure aus wässrigen Lösungen, wie sie bei der Hydrolyse von Methylformiat anfallen.

Aus «Ullmanns Encyklopädie der technischen Chemie», 4. Auflage, Band 7, Seite 365, ist es bekannt, Ameisensäure durch Acydolyse von Formamid mit Schwefelsäure herzustellen. Dieses im grosstechnischen Massstab ausgeübte Verfahren hat jedoch den Nachteil, dass man hierbei stöchiometrische Mengen an Ammoniumsulfat als Zwangsanfall erhält.

Trotz dieses Nachteils hat die ebenfalls bekannte (Ullmann cit., Seite 366), auf den ersten Blick wesentlich günstiger erscheinende Hydrolyse von Methylformiat

$$HCOOCH_3 + H_2O \rightleftharpoons HCOOH + CH_3OH$$

bisher keinen Eingang in die Technik gefunden, und zwar hauptsächlich wegen der hohen Geschwindigkeit der Rückveresterung, welche durch die katalytisch wirkende starke Ameisensäure bedingt wird. Dementsprechend ungünstig ist das Hydrolysegleichgewicht, in welchem alle vier Komponenten in hohen Anteilen vorliegen.

Eine Gleichgewichtsverschiebung durch destillative Entfernung der gewünschten Verfahrensprodukte ist nicht möglich, weil das Methylformiat (Sdp. 32°C) wesentlich tiefer siedet als Methanol (Sdp. 65°C) und Ameisensäure (Sdp. 101°C).

Eine weitere Schwierigkeit, Ameisensäure auf dem Wege über die Hydrolyse von Methylformiat herzustellen, ergibt sich daraus, dass hierbei zunächst verdünnte wässrige Ameisensäurelösungen anfallen, aus denen reine oder höher konzentrierte Ameisensäure wegen Azeotropbildung mit Wasser nicht ohne weiteres destillativ gewonnen werden kann.

Nach der Lehre der US-PS 2 160 064 nutzt man die bei unterschiedlichem Druck unterschiedliche Zusammensetzung dieser Azeotrope dazu aus, reine oder konzentrierte Ameisensäure zu gewinnen. Hierbei zerlegt man die verdünnte Säure zunächst bei relativ hohem Druck in Wasser als Kopfprodukt und ein an Ameisensäure reiches Azeotrop als Sumpfprodukt. Bei der Destillation dieses Azeotropes in einer zweiten, bei relativ geringerem Druck betriebenen Kolonne erhält man dann als Kopfprodukt Ameisensäure und als Sumpfprodukt ein Azeotrop, welches weniger Ameisensäure enthält als das der ersten Destillationsstufe. Das Azeotrop der zweiten Stufe wird sodann wieder in die erste Stufe zurückgegeben. Dieses Verfahren ist jedoch technisch nicht brauchbar. Ist die Druckdifferenz gross, gelingt zwar die Trennung, aber nur auf Kosten eines wirtschaftlich nicht tragbaren Verfahrensaufwandes, und ist sie gering, so fällt die Trennleistung auf Null ab. Ausserdem muss das gesamte Wasser unter hohem Energieverbrauch destillativ entfernt werden.

Auch die bisherigen Versuche, die Ameisensäure mit Hilfe eines Extraktionsmittels aus dem Gleichgewicht zu entfernen, konnten nicht befriedigen.

So ist es aus der DE-A 27 44 313 bekannt, die Hydrolyse in Gegenwart einer organischen Base vorzunehmen, wobei sich ein Addukt aus der Ameisensäure und der Base bildet, von welchem sich die übrigen Reaktionspartner leicht abdestillieren lassen. Nachteilig hierbei ist jedoch nicht nur der insgesamt zu hohe Destillationsaufwand, sondern auch der Umstand, dass die Spaltung des Addukts relativ scharfe Destillationsbedingungen erfordert, bei denen die Ameisensäure und die Base sich bereits zu zersetzen beginnen. Infolgedessen ist es erforderlich, die Ameisensäure nochmals zu destillieren. Mit Hilfe dieses Verfahrens lässt sich reine Ameisensäure über Methylformiat somit nicht wirtschaftlich herstellen.

Nach der Lehre der DE-A 25 45 658 unterwirft man eine wässrige Ameisensäure, wie man sie nach destillativer Abtrennung des Methylformiates und des Methanols aus dem Hydrolysegemisch erhält, einer Flüssig-flüssig-Extraktion mit N-Di-n-butylformamid oder ähnlichen Carbonsäureamiden. Für sich allein stellt dieses Verfahren jedoch keine Lösung des Problems der technisch-wirtschaftlichen Gewinnung von Ameisensäure dar, weil man im Anschluss an die Extraktion das in der Extraktphase noch vorhandene Wasser abtrennen muss. Die Abtrennung des Wassers, sei es durch übliche Fraktionierung oder sei es mittels zusätzlicher Extraktionsstufen, ist aber energieaufwendig, und es ist der DE-OS nicht zu entnehmen, wie dieser Nachteil vermieden werden könnte.

Nach dem Verfahren der DE-A 28 59 991 werden die Hydrolyse des Methylformiats und die Entwässerung der Ameisensäure in einer einzigen Reaktionskolonne vorgenommen.

Die Energiebilanz aller bekannten Verfahren ist nicht zuletzt auch deswegen unbefriedigend, weil das gesamte Wasser oder zumindest ein Grossteil davon ständig unter Verdampfung und Kondensation im Kreise geführt werden muss. Wegen der hohen Verdampfungswärme des Wassers wird hier also besonders viel Energie vernichtet.

Aus diesem Grunde war man stets bestrebt, den Wasseranteil möglichst gering zu halten, d.h. nicht wesentlich mehr als die stöchiometrisch für die Hydrolyse erforderliche Menge zu verwenden. Dies schloss die Möglichkeit aus, das Hydrolysegleichgewicht durch einen Wasserüberschuss zugunsten der Ameisensäure zu verbessern.

Der Erfindung lag daher die wirtschaftlichere Gewinnung wasserfreier oder weitgehend wasserfreier Ameisensäure aus den Hydrolysegemischen von Methylformiat als Aufgabe zugrunde.

Es wurde gefunden, dass man wasserfreie oder weitgehend wasserfreie Ameisensäure durch Hydrolyse von Methylformiat erhält, wenn man

a) Methylformiat der Hydrolyse unterwirft,

b) vom erhaltenen Hydrolysegemisch das Methanol sowie das überschüssige Methylformiat abdestilliert,

c) das aus Ameisensäure und Wasser bestehende Sumpfprodukt der Destillation (b) in einer Flüssig-flüssig-Extraktion mit einem hauptsächlich die Ameisensäure aufnehmenden Extraktionsmittel extrahiert,

d) die hierbei erhaltene, aus Ameisensäure, dem Extraktionsmittel und einem Teil des Wassers bestehende Extraktphase einer Destillation unterwirft.

e) das bei dieser Destillation erhältliche, aus der Gesamtmenge oder einer Teilmenge des in die Destillation eingeführten Wassers und einem Teil der Ameisensäure bestehende Kopfprodukt dampfförmig in den unteren Teil der Destillationskolonne der Stufe (b) zurückführt,

f) das aus dem Extraktionsmittel, gegebenenfalls einer Teilmenge des Wassers und dem Grossteil der Ameisensäure bestehende Sumpfprodukt der Destillationsstufe (d) destillativ in wasserfreie bzw. weitgehend wasserfreie Ameisensäure und das Extraktionsmittel auftrennt und

g) das die Stufe (f) verlassende Extraktionsmittel in den Verfahrensgang zurückführt.

Es wurde weiterhin gefunden, dass es bei diesem Verfahren besonders zweckmässig ist,

h) die Destillationsschritte (b) und (d) in einer einzigen Kolonne vorzunehmen, welche die Funktionen der Kolonnen dieser Schritte erfüllt, und/oder

i) das für die Hydrolyse benötigte Wasser dampfförmig in den unteren Teil der Kolonne des Schrittes (b) einzubringen und/oder

k) Methylformiat und Wasser bei der Hydrolyse (a) im Molverhältnis 1:2 bis 1:10 einzusetzen und/oder

l) als Extraktionsmittel ein Carbonsäureamid der allgemeinen Formel I

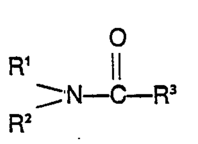 (I)

zu verwenden, in der die Reste $R^1$ und $R^2$ Alkyl-, Cycloalkyl-, Aryl- oder Aralkylgruppen oder gemeinsam eine 1,4- oder 1,5-Alkylengruppe mit jeweils 1 bis 8 C-Atomen mit der Massgabe bedeuten, dass die Summe der C-Atome von $R^1$ und $R^2$ 7 bis 14 beträgt und dass nur einer der Reste eine Arylgruppe ist, und in der $R^3$ für — vorzugsweise Wasserstoff — oder eine $C_1$-$C_4$-Alkylgruppe steht und/oder

m) im Falle der Verwendung eines Extraktionsmittels (l) die Hydrolyse (a) in Gegenwart des Extraktionsmittels vorzunehmen.

Das erfindungsgemässe Verfahren sei anhand der Zeichnungen 1 und 2 erläutert, und zwar zur Veranschaulichung der durch den Verbund ermöglichten Vorteile jeweils als Teil der Gesamtsynthese von Ameisensäure aus Kohlenmonoxid und Wasser gemäss den Reaktionen

$$CO + CH_3\text{-}OH \rightarrow CH_3\text{-}O\text{-}CO\text{-}H$$
$$CH_3\text{-}O\text{-}CO\text{-}H + H_2O \rightarrow CH_3\text{-}OH + H\text{-}CO\text{-}OH$$
$$\text{Brutto: } CO + H_2O \rightarrow H\text{-}CO\text{-}OH$$

Dass auch die Hilfsstoffe wie Methanol oder das Extraktionsmittel einem gewissen Verbrauch unterliegen, ist selbstverständlich und bedarf deshalb keiner näheren Erläuterung.

Zeichnung 1 zeigt das in den Apparaturen H, D1, E, D2 und D3 ausgeübte Verfahren in der allgemeinen Form gemäss den Verfahrensmerkmalen (a) bis (g).

Das den Hydrolysereaktor H verlassende Gemisch aus Methylformiat (MF), Wasser (W), Ameisensäure (AS) und Methanol (Me) gelangt zunächst in die Destillationskolonne D1, in welcher gemäss Verfahrensschritt (b) Methylformiat und Methanol von der wässrigen Ameisensäure abdestilliert werden. Im Verbund der Gesamtsynthese arbeitet man hierbei zweckmässigerweise so, dass man das Methylformiat, welches nicht vollständig methanolfrei zu sein braucht, über Kopf abzieht und in den Hydrolysereaktor H zurückführt und das Methanol, welches noch etwas Methylformiat enthalten kann, als höher siedenden Seitenstrom entnimmt und in den Synthesereaktor R zurückführt.

Das aus Ameisensäure und Wasser bestehende Sumpfprodukt von D1 gelangt in die Flüssig-flüssig-Extraktionskolonne E, wo es gemäss Verfahrensschritt (c) mittels des Extraktionsmittels (Ex) im Gegenstrom weitgehend vom Wasser befreit wird. In den meisten Fällen sind Wasser und Ameisensäure schwerer als das Extraktionsmittel und die hauptsächlich aus dem Extraktionsmittel, Ameisensäure und Wasser bestehende Extraktphase, so dass sich daraus die Zugabe- und die Entnahmestellen bei E ergeben. Verhält es sich umgekehrt, wären Zugabe- und Entnahmestellen entsprechend zu vertauschen. Das Wasser, welches E im Normalfall unten verlässt, wird zweckmässigerweise nach H zurückgeführt, wogegen die stets noch wasserhaltige Extraktphase in die Destillationskolonne D2 gelangt.

Das Kopfsprodukt dieser Destillation (d), welches hauptsächlich aus Wasser und geringen Anteilen Ameisensäure besteht, wird gemäss Verfahrensschritt (e) dampfförmig nach D1 zurückgeführt, wo es einen Teil oder die Gesamtmenge der dort benötigten Energie liefert. Das aus Ameisensäure und dem Extraktionsmittel bestehende Sumpfprodukt von D2 wird gemäss Verfahrensschritt (f) in D3 in seine Komponenten zerlegt, wonach das Extraktionsmittel gemäss Verfahrenschritt (g) nach E zurückgeführt wird. Will man von der besonderen Ausführungsform (m) Gebrauch machen, so führt man einen Teil des Extraktionsmittels in den Hydrolysereaktor H zurück, wie durch die gestrichelt eingezeichnete Leitung veranschaulicht wird. In diesem Fall enthält der Produktstrom von H über D1 nach E zusätzlich das Extraktionsmittel.

Verringert man die Trennwirkung in D2, so erhält man als Sumpfprodukt von D2 ein wasserhaltiges Gemisch, welches in D3 statt wasserfreier eine entsprechend wasserhaltige Ameisensäure liefert, wie es für viele Zwecke genügt.

Das für die Hydrolyse benötigte Wasser kann flüssig zugeführt werden. Steht jedoch ohnehin Industriedampf zur Verfügung, so setzt man das Wasser vorzugsweise dampfförmig ein, weil dadurch ein Teil des Energiebedarfs gedeckt wird. Beispielsweise kann der Wasserdampf in den Strom D2-D1 aufgenommen und in den unteren Teil von D1 geführt werden.

Zeichnung 2 veranschaulicht die räumliche Zusammenlegung der in den Destillationskolonnen D1 und D2 vorgenommenen Verfahrensschritte (b) und (d) in eine einzige Gesamtkolonne G gemäss der bevorzugten Ausführungsform (h). Wie man erkennt, besteht der Unterschied gegenüber dem allgemeinen Verfahrensschema lediglich darin, dass die Leitung «AS/W, Dampf» von D2 nach D1 entfällt, da D1 und D2 kurzgeschlossen sind. Nähere Erläuterungen zu Zeichnung 2 sind daher entbehrlich.

Im einzelnen empfehlen sich für die Verfahrensschritte (a) bis (m) sowie deren apparativen Ausgestaltungen die nachstehenden Ausführungsformen.

Verfahrensschritt (a)

Die Hydrolyse (a) wird allgemein wie üblich bei 80 bis 150°C vorgenommen. Die speziellen Ausführungsformen (k) und (m) werden weiter unten erläutert.

Verfahrensschritt (b)

Die Destillation des Hydrolysegemisches kann prinzipiell unter beliebigem Druck (etwa von 0,5 bis 2 bar), vorgenommen werden, jedoch empfiehlt sich im allgemeinen das Arbeiten unter Normaldruck. In diesem Fall beträgt die Temperatur im Kolonnensumpf etwa 110°C und am Kolonnenkopf etwa 30 bis 40°C. Das Hydrolysegemisch wird zweckmässigerweise im Temperaturbereich von 80 bis 150°C zugegeben, und das Methanol entnimmt man flüssig im Bereich von 55 bis 65°C. Eine zufriedenstellende Trennung des Gemisches in Methylformiat und Methanol einerseits und die wässrige Ameisensäure andererseits ist bereits mit 25 theoretischen Böden möglich, wogegen eine höhere Bodenzahl als 60 keine nennenswerten Vorteile mehr bringt. Bevorzugt wird eine theoretische Bodenzahl von 35 bis 45. Die Bauart der Kolonne D1 kann beliebig sein, besonders empfehlen sich jedoch Siebboden- oder Füllkörper-Kolonne, weil deren Fertigung aus korrosionsbeständigen Materialien relativ einfach ist, so dass sie billiger sind als Kolonnen sonstiger Bauart.

Das Methanol und das Methylformiat führt man zweckmässigerweise in den Synthesereaktor R bzw. den Hydrolysereaktor H zurück, jedoch ist dies kein Kriterium des erfindungsgemässen Verfahrens. Da geringe Mengen Methylformiat bei der Synthese ebensowenig stören wie geringe Mengen Methanol bei der Hydrolyse, braucht die destillative Trennung von Methylformiat und Methanol nicht vollständig zu sein. Im allgemeinen genügt eine jeweilige Reinheit von 90 Gew.-%.

Verfahrensschritt (c)

Die Flüssig-flüssig-Extraktion der Ameisensäure aus ihrer wässrigen Lösung mittels eines Extraktionsmittels wird vorzugsweise bei Normaldruck und bei Temperaturen von 60 bis 120, insbesondere 70 bis 90°C nach den hierfür üblichen Techniken im Gegenstrom vorgenommen. Je nach Art des Extraktionsmittels benötigt man in der Regel Trennapparate mit 1 bis 12 theoretischen Trennstufen, wobei im Falle nur einer Trennstufe die Kolonne zu einem Abscheider entartet. In den meisten Fällen erzielt man mit 4 bis 6 theoretischen Trennstufen befriedigende Ergebnisse. Von der Bauart des Trennapparates ist das Verfahren grundsätzlich nicht abhängig, d.h. man kann Siebboden- oder Füllkörperkolonnen mit oder ohne Pulsation verwenden sowie Apparate mit rotierenden Einbauten oder Mixer-Settler-Batterien.

Die Art des Extraktionsmittels ist nicht erfindungskritisch, vielmehr eignen sich alle Flüssigkeiten, in denen sich die Ameisensäure löst und die mit Wasser nicht oder wenig mischbar sind. Diese Veraussetzung allein genügt für technische Zwecke meistens jedoch noch nicht. Verwendet man beispielsweise ein für polare hydrophile Verbindungen schwach affines Extraktionsmittel wie Benzol oder einen chlorierten Kohlenwasserstoff, so enthält die Extraktphase zwar wenig Wasser und relativ viel Ameisensäure, absolut jedoch auch nur wenig Ameisensäure. In diesem Falle müsste man also zur Erzielung ausreichender Produktionskapazitäten unverhältnismässig grosse Mengen des Extraktionsmittels mit grossem apparativem und energetischem Aufwand im Kreise führen.

Ist andererseits die Affinität des Extraktionsmittels zur Ameisensäure sehr stark, so gelangt wegen der grossen Affinität des Wassers zur Ameisensäure meistens auch viel Wasser in die Extraktphase. Hierin liegt ebenfalls ein Nachteil, der jedoch bei dem vorliegenden erfindungsgemässen Verfahren nicht so stark ins Gewicht fällt. Der wirtschaftliche Kompromiss zwischen den Nachteilen der selektiven, dafür aber kapazitätsarmen Extraktionsmittel und der weniger selektiven, jedoch aufnahmefähigen

Extraktionsmittel liegt daher eher auf der Seite der letztgenannten Mittel.

Die Wirkung der Extraktionsmittel kann auf einem rein physikalischen Lösungsvorgang oder auf einer chemischen Absorption unter Bildung von thermisch leicht zersetzlichen salzartigen Verbindungen oder Wasserstoffbrücken-Addukten beruhen. Trifft letzteres zu, setzt man die Extraktionsmittel vorzugsweise in etwa äquimolaren bis leicht überschüssigen Mengen zur Ameisensäure ein, also im Molverhältnis Ex:AS = 1:1 bis 3:1. Im Falle eines Lösevorgangs liegt das Volumenverhältnis Ex:AS im allgemeinen zwischen 2:1 bis 5:1. Für Mischformen zwischen Lösungsextraktion und chemischer Absorption gelten entsprechende Durchschnittswerte zwischen den beiden genannten Bereichen.

Als Extraktionsmittel besonders gut geeignet haben sich die mit einer gewissen chemischen Affinität wirkenden Carbonsäureamide der allgemeinen Formel I

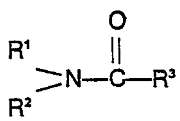 (I)

erwiesen, in der die Reste $R^1$ und $R^2$ Alkyl-, Cycloalkyl-, Aryl- oder Aralkylgruppen oder gemeinsam eine 1,4- oder 1,5-Alkylengruppe mit jeweils 1-8 C-Atomen mit der Massgabe bedeuten, dass die Summe der C-Atome von $R^1$ und $R^2$ 7-14 beträgt und dass nur einer der Reste eine Arylgruppe ist, und in der $R^3$ für — vorzugsweise Wasserstoff — oder eine $C_1$-$C_4$-Alkylgruppe steht.

Derartige Extraktionsmittel sind vor allem N-Di-n-butylformamid und daneben N-Di-n-butylacetamid, N-Methyl-N-2-heptylformamid, N-n-Butyl-N-2-äthylhexylformamid, N-n-Butyl-N-cyclohexylformamid, N-Äthylformanilid sowie Gemische dieser Verbindungen. Wegen der Möglichkeit der Umamidierung im Falle der Amide höherer Säuren, welche zu einer Freisetzung dieser Säuren durch die Ameisensäure führen kann, sind die Formamide allgemein zu bevorzugen.

Weitere geeignete Extraktionsmittel sind u.a. Diisopropyläther, Methylisobutylketon, Äthylacetat, Tributylphosphat und Butandiolformiat.

Als Raffinatphase erhält man in allen Fällen fast ausschliesslich Wasser und daneben etwas Ameisensäure und geringe Mengen des Extraktionsmittels. Die Begleitstoffe stören jedoch nicht, da sie im Verfahrenskreislauf wieder in die Extraktionsstufe zurückgelangen.

Die Extraktphase besteht hauptsächlich aus nahezu der gesamten Ameisensäure, nahezu dem gesamten Extraktionsmittel und etwa 30 bis 60 Gew.-% Wasser, bezogen auf die Ameisensäure.

Verfahrensschritt (d)

Die aus E stammende Extraktphase wird in der Kolonne D2 destillativ in eine Flüssigphase, die aus der Ameisensäure, dem Extraktionsmittel und gegebenenfalls — sofern man eine wasserhaltige Ameisensäure gewinnen will — Wasser besteht, sowie in eine Dampfphase aus Wasser und geringen Mengen Ameisensäure zerlegt. Da hier ein Extraktionsmittel zugegen ist, welches die neben dem Wasser z.T. ebenfalls verdampfende Ameisensäure in die Flüssigphase aufnimmt, kann der Verfahrensschritt (d) auch als Extraktivdestillation bezeichnet werden.

Die Sumpftemperatur bei dieser Destillation beträgt vorzugsweise 140 bis 180°C. Ein vollständiger Trenneffekt, bei dem also kein Wasser in den Sumpf gelangt, wird ab 5 theoretischen Böden erzielt. Für den Fall der Gewinnung einer 90 gew-%igen wässrigen Ameisensäure sind allerdings ebenfalls noch 5 theoretische Böden erforderlich und erst bei noch geringeren Konzentrationen kommt man mit 4 bis 3 Böden aus. Wie bei der Kolonne D1 kommt es auf die Bauart der Kolonne D2 nicht an, so dass hier das gleiche gilt wie für die Kolonne D1.

Verfahrensschritt (e)

Die Rückführung des Ameisensäuren-Wassergemisches von D2 nach D1 in Dampfform ist ein für die Erfindung besonders wesentliches Merkmal. Im Vergleich zu den vorbekannten Verfahren bedeutet es, dass unabhängig von der Gesamtwassermenge nur diejenige Menge des Wassers verdampft zu werden braucht, die bei der Extraktion in die Extraktphase gelangt und dass die Verdampfungsenergie unmittelbar in D1 wieder nutzbar gemacht werden kann. Da diese Energie ohnehin aufgewendet werden müsste, verläuft die Extraktivdestillation in D2 weitgehend energiefrei.

Gegenüber herkömmlichen Verfahrensweisen beträgt die Energieersparnis mindestens 5 Gigajoule pro t reiner Ameisensäure.

Verfahrensschritte (f) und (g)

Diese Verfahrensschritte entsprechen der üblichen Technik und tragen somit zum Wesen der Erfindung nichts bei. Ihre gesonderte Erwähnung diente lediglich zur Vervollständigung der erfindungsgemässen Lehre. Es sei lediglich angemerkt, dass man die Kolonne D3 zweckmässigerweise unter vermindertem Druck und entsprechend niedrigen Kopftemperaturen — etwa 50 bis 300 mbar und 30 bis 60°C — betreibt, damit die Ameisensäure sich nicht zersetzt.

Verfahrensmerkmal (h)

Diese erfinderische Ausgestaltung des Verfahrens entspricht den Schritten (b) und (d), wenn man die Kolonnen D1 und D2 übereinander zu einer Gesamtkolonne G anordnet und somit unter Wegfall der Leitung D2-D1 kurzschliesst. Die Vorteile dieser besonders eleganten Verfahrensauslegung, für welche im übrigen die Angaben zu den Verfahrensschritten (b) und (d) gelten, liegen auf der Hand, so dass nähere Erläuterungen sich hierzu erübrigen.

Verfahrensmerkmal (i)

Hier handelt es sich ebenfalls um eine erfinderische Ausführungsform, die den Zweck hat, mit dem ohnehin für die Hydrolyse benötigten Wasser gleichzeitig noch Wärmeenergie in Form von Dampf in das Verfahren einzuführen, sofern Industriedampf zur Verfügung steht. Trifft letzteres nicht zu, so kann man das Frischwasser an praktisch beliebiger Stelle auch flüssig in den Hydrolysreaktor einbringen.

Verfahrensmerkmal (k)

Diese Verfahrensweise ist von besonderem erfinderischem Rang, denn es ist nun in Kombination mit den übrigen Verfahrensmerkmalen wirtschaftlich möglich, von solchen Hydrolysegemischen auszugehen, die ihrerseits aus Methylformiat und molar überschüssigen Mengen Wasser hergestellt wurden. Hierdurch wird das Gleichgewicht zugunsten der Ameisensäure verschoben, so dass sich der Destillationsaufwand für das nicht umgesetzte Methylformiat ermässigt und die Kapazität der Ameisensäuregewinnung erhöht wird. Durch die grösseren Wassermengen werden allenfalls grössere Apparaturen erforderlich, jedoch wird der Energieverbrauch praktisch nicht erhöht, weil das zusätzliche Wasser nicht verdampft zu werden braucht, da es im Flüssigkreislauf bleibt.

Verfahrensmerkmal l)

Dieses Merkmal betrifft die besonders gute Eignung der Extraktionsmittel (I), vor allem die des Di-n-butylformamid. Nähere Erläuterungen siehe unter Merkmal (c).

Verfahrensmerkmal (m)

Es wurde gefunden, dass sich das Gleichgewicht im Hydrolyseschritt (a) zugunsten der Ameisensäure verschieben lässt, wenn man die Hydrolyse in Gegenwart eines der Extraktionsmittel (I), darunter besonders von N-Di-n-butylformamid, vornimmt. Vorzugsweise setzt man hierbei 0,5 bis 2 mol (I) pro Mol Methylformiat ein. Die bevorzugte Wassermenge beträgt in diesem Falle 0,5 bis 2 mol pro Mol Methylformiat. Bei dieser Verfahrensweise lässt sich somit die Wassermenge im Wasserkreislauf erheblich verringern. Hierdurch erhöht sich der Wirkungsgrad bei der Extraktion, so dass man besonders in diesem Fall bereits mit einer Trennstufe auskommt, d.h. dass ein einfacher Abscheider als Trennapparat genügt. Da man deswegen weniger Extraktionsmittel für die Extraktion benötigt, empfiehlt sich hier vor allem die Verfahrensweise (h), denn hierdurch gelangt nur soviel Extraktionsmittel von D3 nach E1 wie man zur Extraktion benötigt. Bei der Grundverfahrensweise, die statt des kombinierten Schrittes (h) die Einzelschritte (b) und (d) umfasst, gelangt hingegen die Gesamtmenge des Extraktionsmittels nach E, weswegen E entsprechend grösser dimensioniert werden muss. Energetisch ergeben sich hierdurch aber keine Nachteile.

Sieht man von verfahrenstechnisch bedingten geringen Verlusten an Einsatz- und Hilfsstoffen ab, braucht man bei der Ameisensäure-Gesamtsynthese nur von Wasser und Kohlenmonoxid auszugehen. Durch geringfügige Abänderung der Verfahrensbedingungen lassen sich in einundderselben Anlage alle technisch gebräuchlichen Ameisensäurequalitäten von etwa 75 gew-%iger bis praktisch 100%iger Säure herstellen.

Beispiel 1

Dieses Beispiel wurde in einer Versuchsapparatur gemäss Zeichnung 2, also nach der bevorzugten Verfahrensweise mit dem Merkmal (h) vorgenommen.

Einer Gesamtkolonne G von 5 cm Innendurchmesser, 5 m Höhe und 80 Glockenböden wurden auf Höhe des 35. Bodens (von unten gezählt) bei 120°C stündlich 1734 g eines bei 120°C gewonnenen Hydrolysegemisches aus 16,8 Gew.-% Ameisensäure, 16,4 Gew.-% Methylformiat, 12,3 Gew.-% Methanol und 54,2 Gew.-% Wasser zugeführt. Dies entspricht einem ursprünglichen Methylformiat/Wasser-Verhältnis von 1:5,3 bei der Hydrolyse.

Auf Höhe des 70. Bodens wurden im stationären Betrieb bei 60°C stündlich 167 g Methanol und 25 g Methylformiat flüssig abgezogen, und dem Kolonnenkopf wurden bei 34°C stündlich 313 g Methylformiat und 17 g Methanol entnommen. Die Methylformiatfraktion wurde in den Hydrolysereaktor H geleitet und die Methanolfraktion in den Synthesereaktor R. Auf Höhe des 21. Bodens wurden bei 104°C stündlich 1528 g eines Gemisches aus 268 g Ameisensäure, 28 g N-Di-n-butylformamid und 1232 g Wasser entnommen, welches von oben in eine mit 3 mm-Glasringen gefüllte Pulsationsextraktionskolonne E von 3 m Höhe und 3 cm lichter Weite gegeben wurde. Diese Kolonne hatte 6 theoretische Böden.

Im Gegenstrom wurden stündlich 1287 g N-Di--n-butylformamid zugegeben, das sind 1,54 mol pro Mol Ameisensäure.

Als Extraktphase fiel stündlich ein Gemisch aus 1306 g des Extraktionsmittels, 266 g Ameisensäure und 180 g Wasser an, welches auf Höhe des 20. Bodens zusammen mit 106 g Frischwasser in die Kolonne G geleitet wurde.

Die aus 1052 g Wasser, 6 g Ameisensäure und 5 g des Extraktionsmittels bestehende stündlich anfallende Raffinatphase von E wurde in den Hydrolysereaktor H zurückgeführt.

Vom Sumpf der Kolonne G wurde stündlich bei 170°C ein Gemisch aus 248 g Ameisensäure, 10 g Wasser und 1283 g des Extraktionsmittels

entnommen, welches auf den 10. Boden einer Glockenbodenkolonne D3 mit 30 Böden, 2,5 m Höhe und 5 cm Innendurchmesser aufgegeben wurde.

Die Destillation bei 93 mbar Kopfdruck und einem Rücklaufverhältnis von 1,5 lieferte stündlich 255 g 96 gew.-%ige Ameisensäure. Das noch geringe Mengen Wasser enthaltende Extraktionsmittel wurde in die Extraktionskolonne zurückgeführt.

Beispiel 2

Diese Verfahrensweise glich im Prinzip derjenigen von Beispiel 1, jedoch mit dem wesentlichen Unterschied, dass die Hydrolyse des Methylformiats bei 140°C in Gegenwart von N-Di-n-butylformamid vorgenommen wurde, wodurch sich zur Erzielung der gleichen Ameisensäureausbeute die Mengen der Komponenten in Produktströmen änderten. Die Menge des Hydrolysegemisches betrug in diesem Falle stündlich 2017 g, wobei 12,6 Gew.-% auf das Methylformiat, 9,3 Gew.-% auf das Methanol, 15,3 Gew.-% auf das Wasser und 43,5 Gew.-% auf das Extraktionsmittel entfielen.

Zur Extraktion wurden in diesem Falle stündlich nur 367 g des Extraktionsmittels benötigt. Wie in Beispiel 1 fielen stündlich 245 g 96 gew.-%ige Ameisensäure an.

**Patentansprüche**

1. Verfahren zur Gewinnung von wasserfreier oder weitgehend wasserfreier Ameisensäure durch Hydrolyse von Methylformiat, dadurch gekennzeichnet, dass man

a) Methylformiat der Hydrolyse unterwirft,

b) vom erhaltenen Hydrolysegemisch das Methanol sowie das überschüssige Methylformiat abdestilliert,

c) das aus Ameisensäure und Wasser bestehende Sumpfprodukt der Destillation (b) in einer Flüssig-flüssig-Extraktion mit einem hauptsächlich die Ameisensäure aufnehmenden Extraktionsmittel extrahiert,

d) die hierbei erhaltene, aus Ameisensäure, dem Extraktionsmittel und einem Teil des Wassers bestehende Extraktphase einer Destillation unterwirft,

e) das bei dieser Destillation erhältliche, aus der Gesamtmenge oder einer Teilmenge des in die Destillation eingeführten Wassers und einem Teil der Ameisensäure bestehende Kopfprodukt dampfförmig in den unteren Teil der Destillationskolonne der Stufe (b) zurückführt,

f) das aus dem Extraktionsmittel, gegebenenfalls einer Teilmenge des Wassers und dem Grossteil der Ameisensäure bestehende Sumpfprodukt der Destillationsstufe (d) destillativ in wasserfreie bzw. weitgehend wasserfreie Ameisensäure und das Extraktionsmittel auftrennt und

g) das die Stufe (f) verlassende Extraktionsmittel in den Verfahrensgang zurückführt.

2. Verfahren nach Anspruch 1, dadurch gekennzeichnet, dass man die Destillationsschritte (b) und (d) in einer einzigen Kolonne vornimmt, welche die Funktionen der Kolonnen dieser Schritte erfüllt.

3. Verfahren nach Anspruch 1, dadurch gekennzeichnet, dass man das für die Hydrolyse benötigte Wasser dampfförmig in den unteren Teil der Kolonne des Schrittes (b) einbringt.

4. Verfahren nach Anspruch 2, dadurch gekennzeichnet, dass man das für die Hydrolyse benötigte Wasser dampfförmig in den mittleren Teil der Kolonne einbringt.

5. Verfahren nach den Ansprüchen 1 bis 4, dadurch gekennzeichnet, dass man bei der Hydrolyse (a) Methylformiat und Wasser im Molverhältnis 1:2 bis 1:10 einsetzt.

6. Verfahren nach den Ansprüchen 1 bis 5, dadurch gekennzeichnet, dass man als Extraktionsmittel ein Carbonsäureamid der allgemeinen Formel I

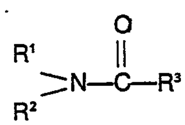

(I)

verwendet, in der die Reste $R^1$ und $R^2$ Alkyl-, Cycloalkyl-, Aryl- oder Aralkylgruppen oder gemeinsam eine 1,4- oder 1,5-Alkylengruppe mit jeweils 1 bis 8 C-Atomen mit der Massgabe bedeuten, dass die Summe der C-Atome von $R^1$ und $R^2$ 7 bis 14 beträgt und dass nur einer der Reste eine Arylgruppe ist, und in der $R^3$ für — vorzugsweise Wasserstoff — oder eine $C_1$-$C_4$-Alkylgruppe steht.

7. Verfahren nach den Ansprüchen 1 bis 6, dadurch gekennzeichnet, dass man im Falle der Verwendung eines Extraktionsmittels (I) die Hydrolyse (a) in Gegenwart des Extraktionsmittels vornimmt.

**Claims**

1. A process for obtaining anhydrous or substantially anhydrous formic acid by hydrolysis of methyl formate, characterized in that

a) methyl formate is hydrolyzed,

b) the methanol and excess methyl formate are distilled from the hydrolysis mixture obtained,

c) the bottom product of distillation (b), consisting of formic acid and water, is extracted, in a liquid-liquid extraction, with an extractant which in the main takes up the formic acid,

d) the resulting extract phase, consisting of formic acid, the extractant and a part of the water, is subjected to distillation,

e) the top product obtained from this distillation and consisting of all or part of the water introduced into the distillation, and part of the formic acid, is recycled, as vapor, to the lower part of the distillation column of stage (b),

f) the bottom product of distillation stage (d),

consisting of the extractant, with or without part of the water, and the greater part of the formic acid, is separated by distillation into anhydrous or substantially anhydrous formic acid and the extractant, and

g) the extractant leaving stage (f) is recycled to the process.

2. A process as claimed in claim 1, characterized in that the distillation steps (b) and (d) are carried out in a single column which performs the functions of the columns of these steps.

3. A process as claimed in claim 1, characterized in that the water required for the hydrolysis is introduced as steam into the lower part of the column of step (b).

4. A process as claimed in claim 2, characterized in that the water required for the hydrolysis is introduced as steam into the middle part of the column.

5. A process as claimed in claims 1 to 4, characterized in that methyl formate and water are employed in a molar ratio of from 1:2 to 1:10 in hydrolysis (a).

6. A process as claimed in claims 1 to 5, characterized in that the extractant employed is a carboxylic acid amide of the general formula I

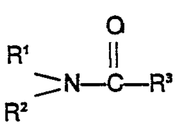

(I)

where $R^1$ and $R^2$ are alkyl, cycloalkyl, aryl or aralkyl or conjointly are 1,4- or 1,5-alkylene, each of 1 to 8 carbon atoms, with the proviso that the sum of the carbon atoms of $R^1$ and $R^2$ is from 7 to 14, and that only one of the radicals is aryl, and where $R^3$ is preferably hydrogen, or $C_1$-$C_4$-alkyl.

7. A process as claimed in claims 1 to 6, characterized in that, if an extractant (I) is used, the hydrolysis (a) is carried out in the presence of the extractant.

## Revendications

1. Procédé de préparation d'acide formique entièrement ou substantiellement anhydre par hydrolyse du formiate de méthyle, caractérisé en ce que:

a) on soumet le formiate de méthyle à une hydrolyse;

b) on sépare du mélange d'hydrolyse obtenu le méthanol et le formiate de méthyle en excès par une distillation;

c) on extrait le produit résiduel de la distillation (b), composé d'acide formique et d'eau, par une extraction liquide — liquide avec un agent d'extraction entraînant principalement l'acide formique;

d) on soumet la phase extraite obtenue, composée d'acide formique, d'agent d'extraction et d'une partie de l'eau, à une distillation;

e) on recycle le produit de tête obtenu dans cette distillation, composé de la totalité ou d'une partie de l'eau introduite dans la distillation et d'une fraction de l'acide formique, à l'état gazeux dans la partie inférieure de la colonne de distillation du stade (b);

f) on sépare le produit résiduel du stade de distillation (d), composé de l'agent d'extraction, éventuellement d'une partie de l'eau et de la majeure partie de l'acide formique, par distillation en un acide formique anhydre ou substantiellement anhydre et en agent d'extraction, et

g) on recycle l'agent d'extraction provenant du stade (f) dans le cycle opératoire.

2. Procédé suivant la revendication 1, caractérisé en ce que les stades de distillation (b) et (d) sont réalisés dans une seule et même colonne, qui remplit les fonctions des colonnes de ces stades.

3. Procédé suivant la revendication 1, caractérisé en ce que l'eau nécessaire à l'hydrolyse est introduite à l'état gazeux dans la partie inférieure de la colonne du stade (b).

4. Procédé suivant la revendication 2, caractérisé en ce que l'eau nécessaire à l'hydrolyse est introduite à l'état gazeux dans la partie médiane de la colonne.

5. Procédé suivant les revendications 1 à 4, caractérisé en ce que, pour l'hydrolyse (a), le formiate de méthyle et l'eau sont mis en œuvre en des proportions molaires dans un rapport de 1:2 à 1:10.

6. Procédé suivant les revendications 1 à 5, caractérisé en ce que l'on emploie comme agent d'extraction un amide d'acide carboxylique de la formule générale I

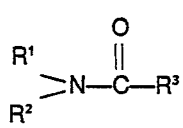

(I)

dans laquelle les restes $R^1$ et $R^2$ désignent des groupes alcolye, cycloalcoyle, aryle ou aralcoyle ou forment ensemble un groupe alcoylène-1,4 ou -1,5 avec 1 à 8 atomes de carbone, avec la condition que la somme des atomes de carbone de $R^1$ et de $R^2$ vaille 7 à 14 et qu'un seul de ces restes représente un groupe aryle, tandis que $R^3$ représente un radical alcoyle en $C_1$ à $C_4$ ou de préférence un atome d'hydrogène.

7. Procédé suivant les revendications 1 à 6, caractérisé en ce que, dans le cas de l'utilisation d'un agent d'extraction (I), l'hydrolyse (a) est réalisée en présence de cet agent d'extraction.

FIG.1

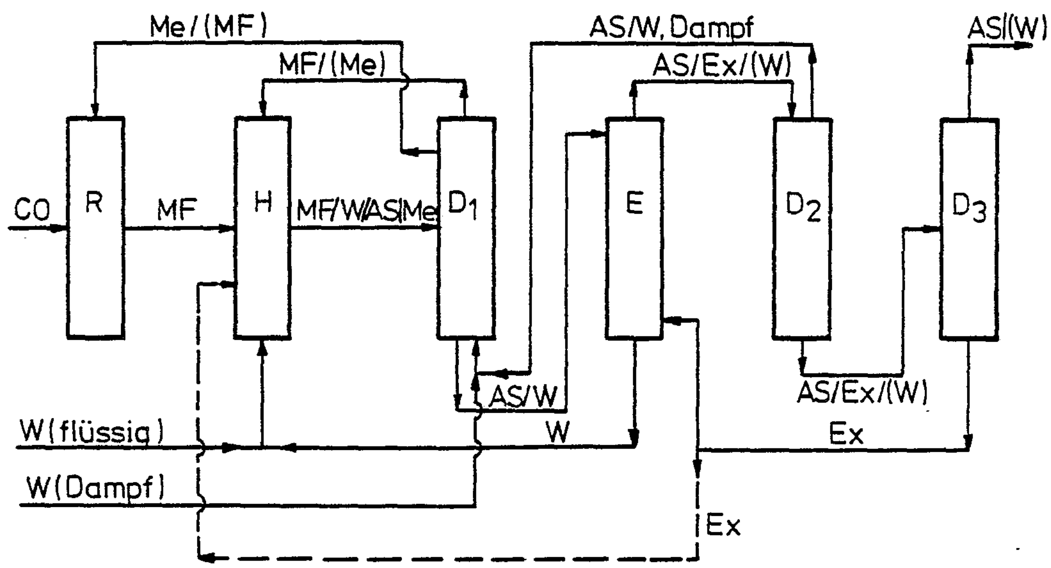


Me = Methanol
MF = Methylformiat
AS = Ameisensäure
W = Wasser
Ex = Extraktionsmittel
( ) = geringere Mengen

FIG.2

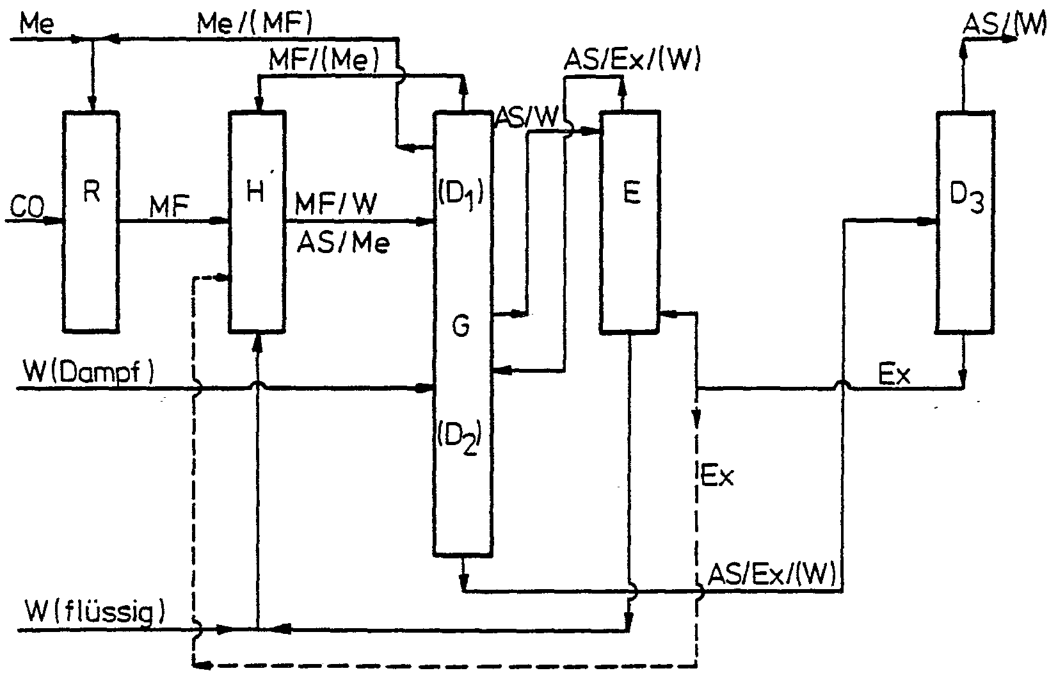


Legende siehe FIG.1